# EUROPÄISCHE PATENTSCHRIFT

(11) **EP 4 103 119 B1**
(45) Veröffentlichungstag und Bekanntmachung des Hinweises auf die Patenterteilung: **16.04.2025**
(21) Anmeldenummer: 21705145.7
(22) Anmeldetag: 10.02.2021
(51) Int. Cl.: A61F 13/05

(54) **WUNDBEHANDLUNGSANORDNUNG**
WOUND TREATMENT ARRANGEMENT
SYSTÈME DE TRAITEMENT DE PLAIES

(30) Priorität: 11.02.2020 DE 202020100710 U
(43) Veröffentlichungstag der Anmeldung: 21.12.2022
(73) Patentinhaber: Lohmann & Rauscher GmbH, 2525 Schönau/Triesting (AT)
(72) Erfinder: LOSKE, Gunnar, 22926 Ahrensburg (DE)
(74) Vertreter: Herrmann, Daniel
(86) Internationale Anmeldenummer: PCT/EP2021/053202
(87) Internationale Veröffentlichungsnummer: WO 2021/160666

(56) Entgegenhaltungen:
- EP-A1- 2 567 681
- WO-A1-2007/051599
- WO-A1-2014/044400
- DE-A1- 102014 216 139
- DE-U1- 202015 000 910
- US-A1- 2018 353 344

## Beschreibung

Die Erfindung betrifft eine Wundbehandlungsanordnung nach dem Oberbegriff des Patentanspruchs 1.

Derartige Wundbehandlungsanordnungen werden insbesondere im Rahmen der oberflächlichen, intraabdominellen, intrathorakalen und/oder intrakorporalen Unterdrucktherapie eingesetzt. Im Rahmen dieser Unterdrucktherapie kann die Drainageeinrichtung zwischen der Wunde und einer okklusiven Folienabdeckung angeordnet werden. In dem so gebildeten, die Drainageeinrichtung enthaltenden Wundraum kann mit Hilfe eines über eine Öffnung in der okklusiven Folienabdeckung und/oder eines an die Drainageeinrichtung angelegten Drainageschlauchs und/oder einer mit der Oberfläche der Drainageeinrichtung in fluidleitendem Kontakt stehendem offenporigen Drainagematerial ein Unterdruck erzeugt werden. Unter dem Einfluss des Unterdrucks erfolgt eine Reinigung der Wunde, bei der das Wundsekret abgesaugt, das interstitielle Ödem reduziert und die Durchblutung verbessert wird.

In der WO 2014/044400 A1 ist eine Wundbehandlungsanordnung nach dem Oberbegriff des Patentanspruchs 1 angegeben. Die WO 2007/051599 offenbart einen Absorptionskörper zur Anbringung an menschliche oder tierische Hautoberflächen mit einer Mischung aus stark osmotisch wirksamen Substanzen und vergleichsweise schwachen oder osmotisch inaktiven Substanzen. In der EP 2567681 A1 ist eine Wundauflage für den Abdominalbereich angegeben. Die DE 102014216139 A1 offenbart ein Wundverbandset zur Behandlung von Wundkavitäten. In der US 2018/0353344 A1 sind Verfahren zum Herstellen von mehrschichtigen Wundmaterialien zum Einsatz bei der Unterdrucktherapie angegeben.

Es hat sich allerdings gezeigt, dass der Einsatz bekannter Wundbehandlungsanordnungen der eingangs angegebenen Art im Rahmen der Unterdrucktherapie in einigen Fällen zu einem nicht befriedigenden Heilungserfolg führt. Angesichts dieser Probleme im Stand der Technik liegt der Erfindung die Aufgabe zugrunde, eine Wundbehandlungsanordnung bereitzustellen, mit der ein zufriedenstellender Therapieerfolg gefördert werden kann.

Erfindungsgemäß wird diese Aufgabe durch die im kennzeichnenden Teil des Patentanspruchs 1 angegebene Weiterbildung der bekannten Wundbehandlungsanordnungen gelöst.

Dabei geht die Erfindung auf die Erkenntnis zurück, dass die Unterdrucktherapie auch bei Behandlung nur einer Wunde unterschiedliche Anforderungen an die Drainageeinrichtung stellen kann, deren Nichterfüllung zu einem nur unbefriedigendem Heilungserfolg führt. Des Weiteren geht die Erfindung auf die Erkenntnis zurück, dass unterschiedliche Wunden unterschiedliche Anforderungen an die Drainageeinrichtung im Rahmen der Unterdrucktherapie stellen können, wobei diese Anforderungen sich auch im Verlauf der Unterdrucktherapie selbst verändern können.

Durch die erfindungsgemäße Weiterbildung bekannter Wundbehandlungsanordnungen mit einer eine mehrschichtige Verbundstruktur aufweisenden Drainageeinrichtung können unterschiedliche Materialeigenschaften in der Drainageeinrichtung vereinigt werden, die therapeutisch in der Unterdrucktherapie nutzbar sind und Nachteile einer Drainageeinrichtung, welche nur eine der Eigenschaften besitzt, beseitigt. Die Erfindung kann insbesondere bei der oberflächlichen, intraabdominellen, intrathorakalen und/oder intrakorporalen Unterdrucktherapie angewendet werden. Durch die erfindungsgemäß vorgesehene Drainageeinrichtung mit einer mehrschichtigen Verbundstruktur wird eine differenzierte Unterdrucktherapie ermöglicht und Nachteile durch die Verwendung einzelner Materialien beseitigt.

Bei bevorzugten Ausführungsformen der Erfindung weist die Drainageeinrichtung nur zwei Drainageschichten auf, so dass die den Begrenzungsflächen abgewandte Grenzschicht zwischen den beiden Drainageschichten gebildet ist, die fluidleitend miteinander verbunden sind. Dabei kann die Verbindung der Drainageschichten durch Klebung, Verschweißung, Vernähung oder durch Laserschweißen oder mit Hilfe anderer Verbindungstechniken vorgenommen werden. Wesentlich ist dabei, dass durch die Verbindung der Drainageschichten ein fluidleitender Kontakt zwischen den Drainageschichten erhalten bleibt. Der fluidleitende Kontakt zwischen den Drainageschichten kann durch die offenporigen fluidleitenden Eigenschaften der Drainageschichtung und der Oberfläche der Drainageschichten erreicht werden. Der fluidleitende Kontakt zwischen den Schichten kann auch durch eigene fluidleitende Verbindungen, Öffnungen in der Oberfläche der Schichten, fluidleitende kommunizierende Kanäle hergestellt werden.

Erfindungsgemäß weist die erste Drainageschicht einen offenporigen Schaum, insbesondere Polyurethanschaum, auf. Dabei wird unter dem Begriff offenporig eine Struktur erfasst, bei der wenigstens einige Poren des Schaums untereinander fluidleitend verbunden sind. Zum Erhalt einer zufriedenstellenden Drainagewirkung hat es sich als sinnvoll erwiesen, wenn die mittlere Porengröße des offenporigen Schaums 200 µm oder mehr und/oder 800 µm oder weniger beträgt. Dabei kann die Dicke der ersten Drainageschicht in einer sich senkrecht zu der ersten äußeren Begrenzungsfläche erstreckenden Dickenrichtung 1mm oder mehr, insbesondere 2mm oder mehr, besonders bevorzugt 10 mm oder mehr und/oder 30 mm oder weniger betragen. Die Gesamtdicke der Drainageeinrichtung beträgt vorzugsweise 1,5 mm oder mehr, vorzugsweise 5mm oder mehr, insbesondere 15 mm oder mehr und/oder 50 mm oder weniger, vorzugsweise 40 mm oder weniger, insbesondere 35 mm oder weniger.

Die durch den offenporigen Schaum, insbesondere Polyurethanschaum, gebildete erste äußere Begrenzungsfläche besitzt die Eigenschaften eines Schwamms, insbesondere Polyurethanschwamms. Über diese erste Begrenzungsfläche kann eine in der Oberfläche ausgerüstete Drainageeinheit nach Anlage eines Unterdruckes, ein Wunddebridement, eine hohe Anhaftung auf Gewebe, eine Granulation von Wundoberflächen, eine gesteigerte Durchblutung und ein erosives der Schwammoberfläche entsprechendes Saugmuster im sogausübenden Kontakt der Wundoberfläche und einem in sogausübenden Kontakt stehenden Körpergewebe im Verlauf einer Unterdrucktherapie erzeugen.

Erfindungsgemäß weist die zweite Drainageschicht der im Rahmen der Erfindung als Drainageeinrichtung eingesetzten mehrschichtigen Verbundstruktureine ein- oder mehrlagige Drainagefolie auf, wobei die zweite äußere Begrenzungsfläche eine von einer Mehrzahl von Perforationsöffnungen durchsetzte Folienlage aufweist.

Im Rahmen der Erfindung ist auch an den Einsatz von solchen Ausführungsformen gedacht, bei denen die zweite Drainageschicht aus einer einlagigen perforierten Folie besteht. Erfindungsgemäß hat es sich allerdings als besonders zweckmäßig erwiesen, wenn die zweite Drainageschicht mindestens zwei Folienlagen aufweist, zwischen denen ein Drainageraum gebildet ist und von denen jede eine Mehrzahl von Perforationsöffnungen aufweist, die in den Drainageraum münden. Bei dieser Ausführungsform der Erfindung wird die zweite äußere Begrenzungsfläche von einer ersten perforierten Folienlage gebildet, während die Grenzschicht zu der benachbarten Drainageschicht von der der zweiten Begrenzungsfläche abgewandten zweiten Folienlage gebildet wird, welche ebenfalls perforiert ist und eine fluidleitende Verbindung mit der benachbarten Drainageschicht, insbesondere der ersten Drainageschicht, gewährleistet.

Geeignete mehrlagige Drainagefolien sind in der WO 2010/124844 angegeben. Ähnlich wie bei den in dieser Schrift beschriebenen Drainagefolien kann auch die zweite Drainageschicht einer Drainageeinrichtung einer erfindungsgemäßen Wundbehandlungsanordnung so ausgeführt sein, dass mindestens eine Perforation einer Folienlage sich durch einen ausgehend von dieser Folienlage in Richtung auf die gegenüberliegende innere Begrenzungsfläche der anderen Folienlage erstreckt und einen in den Drainageraum mündenden Kanal bildet, dessen Kanalwand vorzugsweise einstückig mit der Folienlage ausgeführt ist. Dabei kann sich die Querschnittsfläche des Kanals in einer sich senkrecht zur Tiefenrichtung erstreckenden Ebene, ausgehend von der ersten Folienlage in Richtung auf die gegenüberliegende andere Begrenzungsfläche, insbesondere zum Erhalt einer den Eintritt von Körperflüssigkeit in den Drainageraum begünstigenden Kapillarwirkung verringern.

Im Rahmen der Erfindung ist auch an solche Ausführungsformen gedacht, bei denen mindestens eine Folienlage eine Vielzahl von vorzugsweise rasterförmig angeordneten Perforationsöffnungen aufweist, wobei der Abstand zwischen benachbarten Öffnungen 15 mm oder weniger, vorzugsweise 5 mm oder weniger, insbesondere 3 mm oder weniger beträgt. Die Mündungen der in einer Folienlage angeordneten Öffnungen in einer Projektion längs der Tiefenrichtung können zwischen den Mündungen der in der anderen Folienlage angeordneten Perforationsöffnungen angeordnet sein. Die einzelnen Folienlagen können über punktförmige und vorzugsweise in einer Rasteranordnung ausgeführte Befestigungsbereiche miteinander verbunden sein. Diese Befestigungsbereiche können durch eine die einander gegenüber liegenden Begrenzungsflächen der Folienlagen verbindende Materialbrücke gebildet sein.

Mit Blick auf die gewünschte Drainagewirkung der zweiten Drainageschicht hat es sich als besonders günstig erwiesen, wenn mindestens eine Perforationsöffnung eine Fläche von 0,01 mm² oder mehr und/oder 1 mm² oder weniger in einer sich parallel zur zweiten äußeren Begrenzungsfläche erstreckenden Ebene aufweist. Die zweite durch die Drainagefolie gebildete äußere Begrenzungsfläche besitzt die Eigenschaften einer Folie. Sie entfaltetunter Unterdruckausübung eine flächige Drainagewirkung, erzeugt eine nur geringe Anhaftung an der Wunde und auf Körpergewebe und ist hierdurch insbesondere zur Auflage auf oberflächensensible Organe wie zB Intestinum, Peritoneum, Pleura, Gefäße geeignet. Bei der Unterdruckausübung wird auf der Wundoberfläche und in sogkontaktstehendem Gewebe ein ein dem Muster der Perforationsöffnungen entsprechendes Saugmuster erzeugt.

Je nach spezifischer Wundsituation, kann die Unterdrucktherapie unter Einsatz einer Drainageeinrichtung mit einer mehrschichtigen Verbundstruktur im Rahmen der Erfindung nach den speziellen therapeutischen Erfordernissen erfolgen und individuell passend behandelt werden. Je nach den erforderlichen Eigenschaften der Wundoberfläche wird bei der Behandlung ausgewählt, welche der Begrenzungsflächen der mehrschichtigen Verbundstruktur mit der Wunde direkten Saugkontakt haben soll. Soll beispielsweise eine Wunde debridiert werden, dann wird die erste äußere Begrenzungsfläche der mehrschichtigen Verbundstruktur mit der Wunde in Kontakt gebracht, die sich, bei der Verwendung eines grobporigen Polyurethanschaumes, durch stark debridierenden und adhäsive Eigenschaften auszeichnet. Ist die Wunde weitgehend konditioniert und gereinigt, wird die zweite äußere Begrenzungsfläche, die bei der Verwendung einer offenporigen doppellagigen Drainagefolie eine sogausübende aber eine nicht so stark anhaftende und debridierende Oberfläche aufweist, mit der Wunde in Kontakt gebracht.

Bei der oberflächlichen Therapie mit Hilfe einer erfindungsgemäßen Wundbehandlungsanordnung wird die Wunde über der einliegenden Drainageeinrichtung mit einer Okklusionsfolie verschlossen. Dann kann beispielsweise über einen Saugadapter ein Sog an die Wunde angelegt werden. Alternativ oder zusätzlich kann die Wunde auch mit einer einseitig offenporigen Folie verschlossen werden. Die Wunde kann auch mit einem transparenten Unterdruckverband, oder einem Unterdruckpflaster verschlossen werden, welches fluidleitend mit einem unterdruckerzeugenden System verbunden wird.

Vorzugsweise wird bei der abdominellen Therapie die zweite äußere Begrenzungsfläche, das heißt die Folienseite der Drainageeinrichtung in Form der mehrschichtigen Verbundstruktur, auf die intraabdominellen Organe gelegt. Durch die geringere Anhaftung auf dem Gewebe wir das Risiko für iatrogene Verletzungen des Gewebes vermindert. Die im Rahmen der Erfindung eingesetzte Drainageeinrichtung in Form einer mehrschichtigen Verbundstruktur kann auch zusammengerollt oder gefaltet werden, um beispielsweise eine intraabdominelle Region gezielter zu drainieren.

Bei einer bevorzugten Ausführungsform der Erfindung ist die Drainageeinrichtung schneidund/oder teilbar. Dazu kann sie perforiert sein, so dass eine Teilung zum Erhalt vorgegebener Formen und/oder Größen, ggf. durch Zug erfolgen kann. So können Streifen, Rechtecke, runde, ellipsenartige und/oder spiralige Formen erhalten werden. Das kann die Platzierung in Abhängigkeit von den räumlichen Gegebenheiten erleichtern. Zur Sogausübung können die einzelnen Teile in einen direkten Kontakt gebracht werden. Zusätzlich oder alternativ kann die Sogausübung über Überbrückungselemente, wie etwa offenporige Folienelemente, Polyurethanschäume, Drainagen oder dergleichen erfolgen.

Bei der intrathorakalen Anwendung wird wiederum die zweite äußere Begrenzungsfläche, das heißt die Folienseite der Drainageeinrichtung, auf die Pulmo gelegt. Brustwandseitig kann die erste äußere Begrenzungsfläche, das heißt die durch den offenporigen Schaum gebildete äußere Begrenzungsfläche, liegen, die beispielsweise zu einem Debridement des Brustfells führt.

Im Rahmen der Erfindung ist auch daran gedacht, zwei gleichartige äußere Begrenzungsflächen von Drainageeinrichtungen in Form mehrschichtiger Verbundstrukturen aneinanderzulegen oder die Drainageeinrichtung zu falten. Hierdurch kann eine Drainageeinrichtung konstruiert werden, bei der außenseitig entweder nur eine Polyurethanschaumbegrenzungsfläche oder eine Folienbegrenzungsfläche vorgesehen ist, je nach den lokalen Erfordernissen an der zu behandelnden Wunde. Auch in diesem Zusammenhang kann eine Perforation der Drainageeinrichtung zum Zweck der erleichterten Teilung nützlich sein.

Erfindungsgemäß ist vorgesehen, dass die zweite Drainageschicht von mindestens einem Perforationsloch durchsetzt ist, durch das die benachbarte Drainageschicht, insbesondere die erste Drainageschicht, zugänglich ist. Durch diese Ausführungsform der Erfindung entsteht auf der zweiten äußeren Begrenzungsfläche im Bereich des Perforationslochs bzw. der Perforationslöcher ein Oberflächenareal, das die Eigenschaften der anderen Drainageschicht, insbesondere der aus einem offenporigen Schaum, insbesondere Polyurethanschaum, gebildeten Schicht aufweist. Wird an diese Seite, das heißt an die durch den offenporigen Schaum gebildete Drainageschicht, ein Unterdruck angelegt, entsteht im Bereich der zweiten äußeren Begrenzungsfläche, das heißt im Bereich der mit den Perforationslöchern ausgestatteten zweiten Drainageschicht, ein Sogeffekt auf die Wunde, welcher der ersten Drainageschicht entspricht. Hierdurch werden unterschiedlich oberflächenvermittelte Saugeffekte auf die Wunde ausgeübt. Das Saugverhalten und auch die ausgeübte Sogstärke können hierdurch auf der der Wunde zugewandten Oberfläche unregelmäßig gestaltet werden.. Es kann hierbei auch ein unregelmäßige Sogwirkung auf die Wunde entstehen, wobei im Bereich der Perforationslöcher ein stärkerer Sog an die Wunde angelegt werden kann als im Bereich der zwischen den Perforationslöchern verbleibenden Folienbereiche. Durch diese Eigenschaften kann eine inselartige Wundreinigung im Bereich der Perforationslöcher erhalten werden. Es hat sich gezeigt, dass dadurch ein stärkerer Granulationsreiz auf die Wundoberfläche ausgeübt werden kann, der zur stärkeren Ausbildung von Granulationsgewebe führt.

Die oben dargestellte Ausstattung einer Begrenzungsfläche mit Perforationslöchern kann in jeder Materialkombination des schichtweisen Aufbaus der Drainageeinheit vorgenommen werden. Es kann auch ein Polyurethanschaum mit Perforationslöchern versehen sein und die zweite Drainageschicht, welche geschlossen ist, aus einer Folie bestehen. Diese Ausführung hat den Vorteil, dass durch die unterschiedliche Dicke des Schaumes ein zusätzlicher Granulationsreiz auf eine Wunde ausgeübt werden kann.

Wie der vorstehenden Erläuterung der zuletzt beschriebenen Ausführungsform der Erfindung zu entnehmen ist, kann durch Ausbildung der Perforationslöcher unterschiedlichen Anforderungen an die Wundheilung im Bereich nur einer Wunde genügt werden, wenn dafür gesorgt wird, dass dort, wo ein Debridement erforderlich ist, der Polyurethanschaum durch die Perforationslöcher wirksam ist, während dort, wo eine Anhaftung zu vermeiden ist, die Perforationsfolie in Anlage an die Wunde gelangt.

Erfindungsgemäß ist vorgesehen, dass mindestens ein Perforationsloch eine Öffnungsfläche in einer sich parallel zur zweiten Begrenzungsfläche erstreckenden Ebene von 10 mm² oder mehr, insbesondere 25 mm² oder mehr und/oder 1.000 mm² oder weniger, insbesondere 400 mm² oder weniger aufweist. Dabei kann die zweite Drainageschicht zwei oder mehr Perforationslöcher mit unterschiedlichen Öffnungsflächen und/oder-formen aufweisen. Die Perforationslöcher können kreisrund, ellipsenförmig und/oder polygonal ausgeführt sein. Sie können auch andere Konfigurationen haben. Die Perforationslöcher können in einer regelmäßigen Rasterstruktur, in einer unregelmäßigen Struktur, von gleichbleibender Größe oder unterschiedlicher Größe gebildet sein. Im Sinne der Bereitstellung eines unregelmäßigen Sogs im Bereich der Wundoberfläche hat es sich als zweckmäßig erwiesen, wenn die zweite Drainageschicht eine Mehrzahl von Perforationslöchern aufweist und die einander zugewandten Ränder von mindestens zwei Perforationslöchern, insbesondere allen Perforationslöchern, einen Abstand von 1 mm oder mehr, vorzugsweise 5 mm oder mehr aufweisen, wobei der Abstand zweckmäßigerweise 100 mm oder weniger, insbesondere 50 mm oder weniger betragen kann.

Wie der vorstehenden Erläuterung der oberflächlichen Unterdrucktherapie zu entnehmen ist, kann eine erfindungsgemäße Wundbehandlungsanordnung eine Okklusionsfolie zum Herstellen eines die Drainageeinrichtung enthaltenden geschlossenen Wundraums aufweisen. Die Okklusionsfolie kann dabei unabhängig von der Drainageeinrichtung appliziert werden, so dass die Variabilität der erfindungsgemäßen Wundversorgungsanordnung nicht beeinträchtigt wird. Die Okklusion des Wundraumes kann auch mit einer einseitig offenporigen Folie vorgenommen werden. Die erfindungsgemäße Wundbehandlungsanordnung wird, wie der vorstehenden Erläuterung zu entnehmen ist, zweckmäßigerweise im Rahmen der Unterdrucktherapie eingesetzt. Entsprechend weist die erfindungsgemäße Wundversorgungsanordnung vorzugsweis eine Saugeinrichtung zum Erzeugen eines Unterdrucks im Bereich mindestens einer äußeren Begrenzungsfläche der Drainageeinrichtung auf. Die Saugeinrichtung kann einen mit der Drainageeinrichtung, insbesondere der ersten Drainageschicht, fluidleitend verbundenen Drainageschlauch aufweisen, der andererseits mit einer Unterdruckquelle, wie etwa einer Pumpe oder einem Unterdruckreservoir, verbunden ist. Die Sogvermittlung kann auch mit einer anderen Drainageeinrichtung durch direkten Oberflächenkontakt vorgenommen werden.

Für einige Anwendungsfälle hat es sich als zweckmäßig erwiesen, wenn die Drainageeinrichtung bezüglich einer parallel zu einer äußeren Begrenzungsfläche verlaufenden Wickelachse aufgewickelt ist. In ihrer Grundform weist die Drainageeinrichtung erfindungsgemäßer Wundversorgungsanordnungen allerdings zweckmäßigerweise einen bahnförmigen Verlauf auf, wobei die Drainageeinrichtung rechteckig ausgeführt sein kann mit einer Seitenlänge von 1 bis 50 cm.

Bei einigen Ausführungsformen hat es sich weiter als zweckmäßig erwiesen, wenn die zweite Drainageschicht die erste Drainageschicht im Bereich Ihres Rands zumindest abschnittweise in einer parallel zur zweiten äußeren Begrenzungsfläche verlaufenden Richtung überragt.

Wie der vorstehenden Erläuterung erfindungsgemäßer Wundversorgungsanordnungen zu entnehmen ist, betrifft die Erfindung auch die Verwendung einer Drainageeinrichtung mit einer mehrschichtigen Verbundstruktur zur Herstellung einer erfindungsgemäßen Wundbehandlungsanordnung. Dazu kann die erste Drainageschicht mit einem Drainageschlauch verbunden sein und/oder die Drainageeinrichtung mit einem Drainageschlauch, einer okklusiven Folie, einer einseitig offenporigen Folie und/oder einer Unterdruckquelle zu einem Kit zusammengefügt werden.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich Bezug genommen wird, erläutert. In der Zeichnung zeigt:
- Fig. 1: eine Seitenansicht einer zur Herstellung einer Wundversorgungsanordnung einsetzbaren Drainageeinrichtung gemäß einer ersten Ausführungsform der Erfindung,
- Fig. 2: eine Seitenansicht einer zur Herstellung einer Wundversorgungsanordnung geeigneten Drainageeinrichtung gemäß einer zweiten Ausführungsform der Erfindung,
- Fig. 3: eine perspektivische Darstellung einer zur Herstellung einer erfindungsgemäßen Wundversorgungsanordnung geeigneten Drainageeinrichtung gemäß einer dritten Ausführungsform der Erfindung,
- Fig. 4: eine zur Herstellung einer erfindungsgemäßen Wundversorgungsanordnung geeignete Drainageeinrichtung gemäß einer vierten Ausführungsform der Erfindung,
- Fig. 5: eine Schnittdarstellung der Drainageeinrichtung gemäß Fig. **4****,**
- Fig. 6: eine perspektivische Darstellung einer zur Herstellung einer erfindungsgemäßen Wundversorgungsanordnung geeigneten Drainageeinrichtung gemäß einer fünften Ausführungsform der Erfindung,
- Fig. 7: eine Schnittdarstellung der Drainageeinrichtung gemäß Fig. 6,
- Fig. 8: eine weitere Schnittdarstellung der Drainageeinrichtung gemäß Fig. 6, und
- Fig. 9: eine zur Herstellung einer erfindungsgemäßen Wundversorgungsanordnung geeignete Drainageeinrichtung gemäß einer sechsten Ausführungsform der Erfindung.

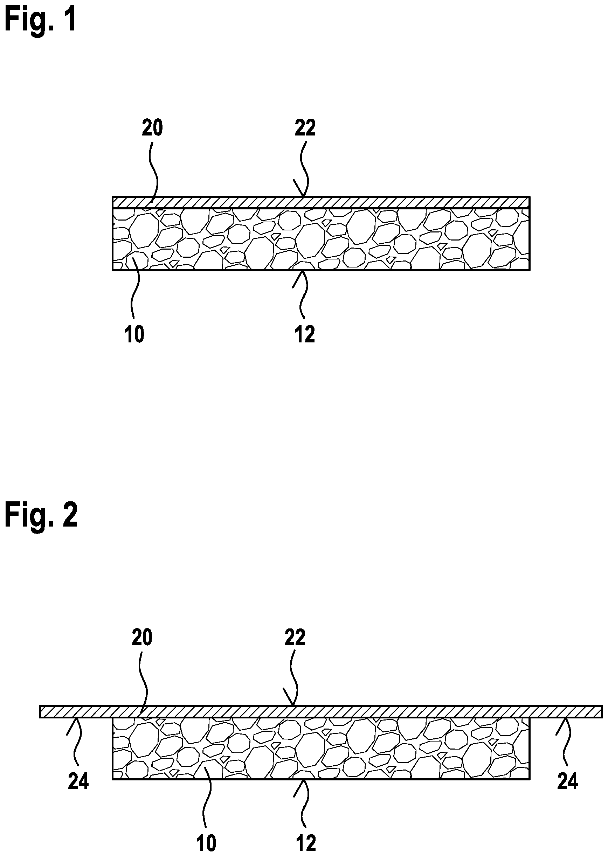

Die in Fig. 1 dargestellte Drainageeinrichtung ist in Form einer zweischichtigen Grundstruktur gebildet. Sie weist eine durch einen offenporigen Polyurethanschaum gebildete erste Drainageschicht 10 und eine durch eine offenporige Folie gebildete zweite Drainageschicht 20 auf. Die zweite Drainageschicht 20 kann als doppellagige offenporige Folie ausgeführt sein, wie in der WO 2010/124844 beschrieben. Sie kann auch einlagig ausgeführt sein. Die erste Drainageschicht 10 ist fluidleitend mit der zweiten Drainageschicht 20 verbunden. So kann durch Anlegen eines Unterdrucks an die erste Drainageschicht 10 auch im Bereich der der ersten Drainageschicht 10 abgewandten äußeren Begrenzungsfläche 22 der zweiten Drainageschicht 20 ein Sog bzw. Unterdruck erzeugt werden, ebenso wie durch Anlegen eines Unterdrucks im Bereich der zweiten Drainageschicht 20 im Bereich der der zweiten Drainageschicht abgewandten Begrenzungsfläche 12 der ersten Drainageschicht 10 ein Unterdruck bzw. Sog erzeugt werden kann.

Die in Fig. 2 dargestellte Ausführungsform unterscheidet sich von der in Fig. 1 dargestellten Ausführungsform im Wesentlichen dadurch, dass die Ränder der zweiten Drainageschicht 20 die Ränder der ersten Drainageschicht 10 überragen. Dadurch wird erreicht, dass die der zweiten Begrenzungsfläche 22 abgewandte Begrenzungsfläche 24 der zweiten Drainageschicht 20 im Bereich der überragenden Ränder freiliegend ausgeführt ist und nicht von der ersten Drainageschicht 10 abgedeckt wird.

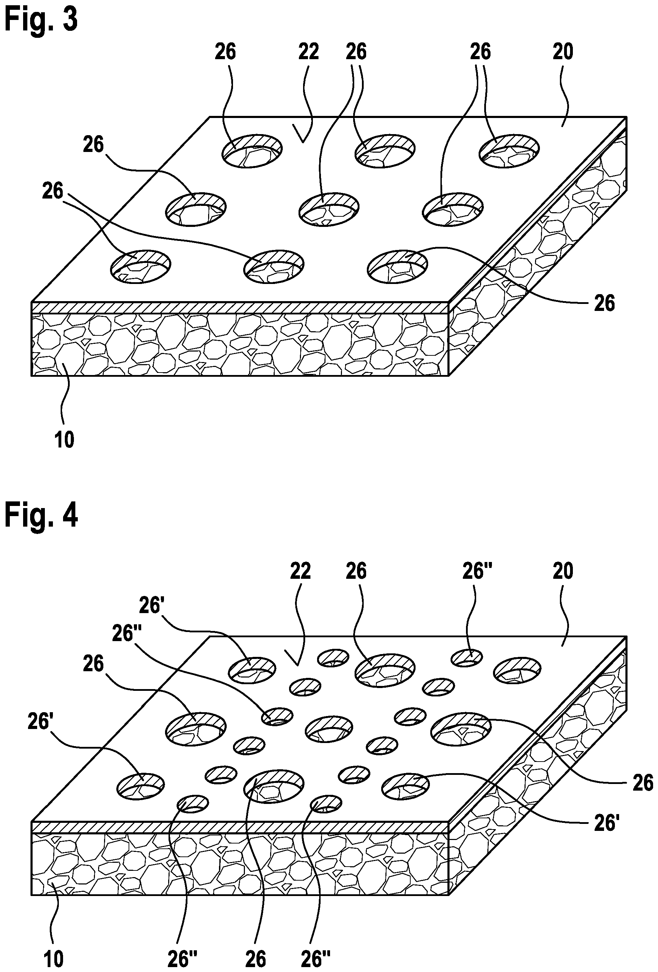

Die in Fig. 3 dargestellte Ausführungsform der Erfindung unterscheidet sich im Wesentlichen dadurch von der anhand der Figuren 1 und 2 erläuterten Ausführungsformen, dass die zweite Drainageschicht 20 von Perforationslöcher 26 durchsetzt ist, durch die die erste Drainageschicht 10 zugänglich ist. So wird erreicht, dass im Bereich der Perforationslöcher 26 ein direkter Kontakt zwischen der ersten Drainageschicht 10 und einer Wunde, auf der die zweite Drainageschicht 20 aufliegt, erzeugt wird und ein den Perforationslöchern entsprechendes Saugmuster im Bereich der Wunde erzeugt werden kann. Wenn die erste Drainageschicht 10 in Form eines offenporigen Polyurethanschaums gebildet ist, entstehen so im Bereich der Perforationslöcher 26 Polyurethanschauminseln, mit denen einerseits ein Debridement und andererseits eine erhöhte Saugwirkung erreichbar ist.

Die in Fig. 4 dargestellte Ausführungsform der Erfindung unterscheidet sich im Wesentlichen dadurch von der anhand der Fig. 3 erläuterten Ausführungsform, dass Perforationslöcher 26, 26', 26" mit unterschiedlichen Größen in der zweiten Drainageschicht 20 vorgesehen sind. Das trägt zur Erzeugung eines gewünschten Saugmusters im Bereich der Wunde bei.

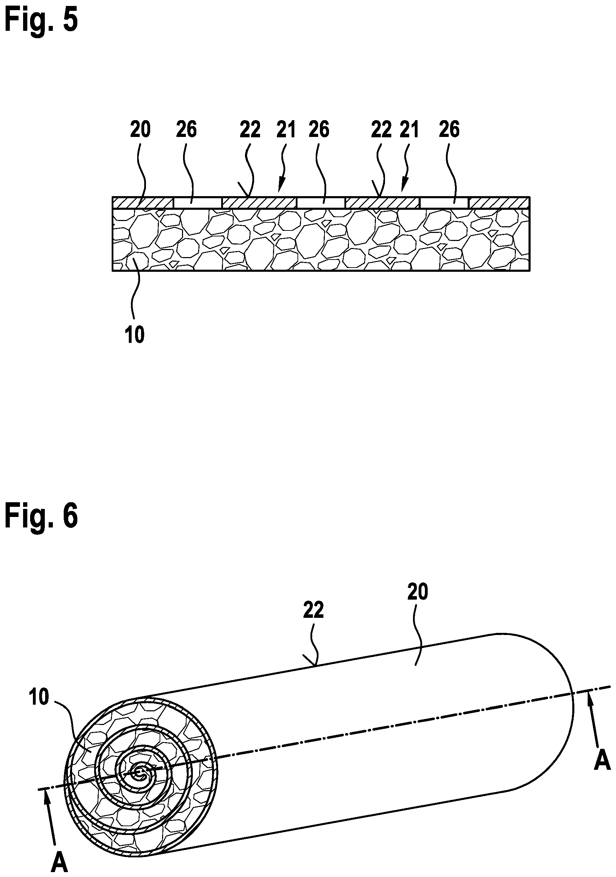

Wie besonders deutlich in Fig. 5 erkennbar ist, sind zwischen den Perforationslöchern 26, in deren Bereich der Polyurethanschaum der ersten Drainageschicht 10 zugänglich ist, folienartige Bereiche 21 der zweiten Drainageschicht 20 vorhanden, die die zweite äußere Begrenzungsfläche 22 der Drainageeinrichtung bilden und an die Wunde anlegbar sind.

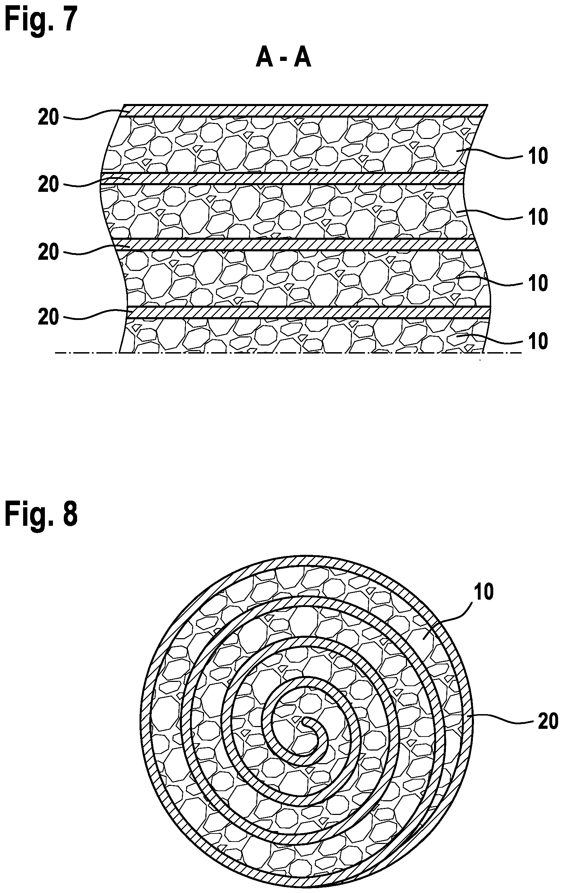

Bei der in den Figuren 6 bis 8 dargestellten Ausführungsform der Erfindung ist die als mehrschichtige Verbundstruktur ausgeführte Drainageeinrichtung über eine parallel zu einer äußeren Begrenzungsfläche der Drainageschichten verlaufende Wickelachse zu einem mehrlagigen Wickel aufgewickelt, wobei die durch die Drainagefolie gebildete zweite äußere Begrenzungsfläche 22 die Außenseite des Wickels bildet.

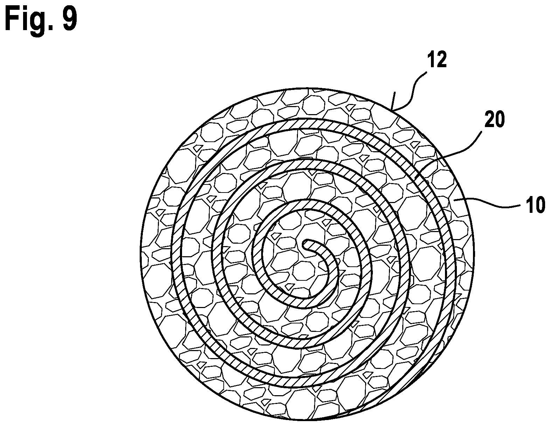

Die in Fig. 9 dargestellte Ausführungsform der Erfindung entspricht im Wesentlichen der anhand der Figuren 6 bis 8 erläuterten Ausführungsform, wobei der Wickel dieser Ausführungsform so ausgeführt ist, dass die durch den offenporigen Polyurethanschaum gebildete erste äußere Begrenzungsfläche der Drainageeinrichtung die Außenseite des Wickels bildet.

Die Erfindung ist nicht auf die anhand der Zeichnung erläuterten Ausführungsformen beschränkt. Vielmehr ist auch an Ausführungsformen gedacht, bei denen die zweite Drainageschicht durch eine einlagige Folie gebildet ist. Die Perforationslöcher können abweichend von der Kreisform auch ellipsenförmig, oval oder polygonal ausgeführt sein. Der Drainageeinrichtung kann ein Drainageschlauch zugeordnet sein, der über einen Port an die zweite Drainageschicht angeschlossen und/oder in die erste Drainageschicht eingesetzt sein kann. Es können auch zwei, drei oder mehr Leitungen zur Herstellung einer fluidleitenden Verbindung zwischen der Drainageeinrichtung und einer Unterdruckquelle vorgesehen sein. Der Drainageeinrichtung kann eine Okklusionsfolie zur Herstellung eines die Drainageeinrichtung enthaltenden Wundraums zugeordnet sein. Der Drainageeinrichtung können auch noch zusätzliche offenporige Drainageelemente, wie etwa ein PU-Schaum, eine Folie, eine Foliendrainage, ein Drainageschlauch oder dergleichen zugeordnet sein. Die Drainageeinrichtung kann im Rahmen der Erfindung frei konfektionierbar ausgeführt sein, indem sie schneidbar ausgestaltet ist. So können vom Operateur Drainageeinrichtungen mit der benötigten Form einfach hergestellt werden, wobei die Drainageeigenschaften durch Positionierung der mehrschichtigen Verbundstruktur in der gewünschten Weise erzielt werden können.

Ferner kann die Drainageeinrichtung einer erfindungsgemäßen Wundversorgungsanordnung auch mit Drainageschläuchen oder anderen fluidleitende Drainagen fluidleitend verbunden sein, über welche der Drainageeinrichtung Spülflüssigkeiten und/oder Medikamente zugeführt werden können.

Mit der Drainageeinrichtung können bei einer entsprechenden Ausführungsform der Erfindung Flüssigkeiten sowohl zugeführt als auch abgesaugt werden .

## Patentansprüche

1. Wundbehandlungsanordnung mit einer zum Ableiten von Fluiden von einem menschlichen odertierischen Körper, insbesondere von einer Wunde ausgelegten Drainageeinrichtung, wobei die Drainageeinrichtung eine mehrschichtige Verbundstruktur mit zwei einander entgegengesetzten, zumindest teilweise fluiddurchlässigen, insbesondere etwa parallel zueinander verlaufenden Begrenzungsflächen aufweist, von denen eine zumindest teilweise von einer Grenzschicht zwischen zwei benachbarten Drainageschichten abgewandten ersten äußeren Begrenzungsfläche einer ersten Drainageschicht mit einer ersten Oberflächenbeschaffenheit und die andere zumindest teilweise von einer Grenzschicht zwischen zwei benachbarten Drainageschichten abgewandten zweiten äußeren Begrenzungsfläche einer zweiten Drainageschicht mit einer sich von der ersten Oberflächenbeschaffenheit unterscheidenden zweiten Oberflächenbeschaffenheit gebildet ist, wobei die Drainageschichten vorzugsweise fluidleitend miteinander verbunden sind, die erste Drainageschicht einen offenporigen Schaum, insbesondere Polyurethanschaum, aufweist und die zweite Drainageschicht eine ein- oder mehrlagige Drainagefolie aufweist, wobei die zweite äußere Begrenzungsfläche von einer von einer Mehrzahl von Perforationsöffnungen durchsetzten Folienlage gebildet ist, **dadurch gekennzeichnet, dass** die zweite Drainageschicht von mindestens einem Perforationsloch durchsetzt ist, durch das die benachbarte Drainageschicht, insbesondere die erste Drainageschicht, zugänglich ist und mindestens ein Perforationsloch eine Öffnungsfläche in einer sich parallel zur zweiten Begrenzungsfläche erstreckenden Ebene von 10 mm² oder mehr, insbesondere 25mm² oder mehr und/oder 1.000 mm² oder weniger, insbesondere 400 mm² oder weniger aufweist.

2. Wundbehandlungsanordnung nach Anspruch 1, **dadurch gekennzeichnet, dass** die mittlere Porengröße des offenporigen Schaums 200 µm oder mehr und/oder 800 µm oder weniger beträgt.

3. Wundbehandlungsanordnung nach einem der vorhergehenden Ansprüche, **dadurch gekennzeichnet, dass** die Dicke der ersten Drainageschicht in einer sich senkrecht zu der ersten äußeren Begrenzungsfläche erstreckenden Dickenrichtung 10 mm oder mehr und/oder 30 mm oder weniger beträgt.

4. Wundbehandlungsanordnung nach einem der vorhergehenden Ansprüche, **dadurch gekennzeichnet, dass** die zweite Drainageschicht zwei Folienlagen aufweist, zwischen denen ein Drainageraum gebildet ist und von denen jede eine Mehrzahl von Perforationsöffnungen aufweist, die in den Drainageraum münden.

5. Wundbehandlungsanordnung nach einem der vorhergehenden Ansprüche, **dadurch gekennzeichnet, dass** die Perforationsöffnungen eine Fläche von 0,01 mm² oder mehr und/oder 1 mm² oder weniger in einer sich parallel zur zweiten äußeren Begrenzungsfläche erstreckenden Ebene aufweisen.

6. Wundbehandlungsanordnung nach einem der vorhergehenden Ansprüche, **dadurch gekennzeichnet, dass** die zweite Drainageschicht mindestens zwei Perforationslöcher mit unterschiedlichen Öffnungsflächen und/oder -formen aufweist.

7. Wundbehandlungsanordnung nach einem der vorhergehenden Ansprüche, **dadurch gekennzeichnet, dass** die zweite Drainageschicht eine Mehrzahl von Perforationslöchern aufweist und die einander zugewandten Ränder von mindestens zwei Perforationslöchern, insbesondere allen Perforationslöchern, einen Abstand von 1 mm oder mehr, vorzugsweise 5 mm oder mehr aufweisen.

8. Wundbehandlungsanordnung nach einem der vorhergehenden Ansprüche, **gekennzeichnet durch** eine Okklusionsfolie zum Herstellen eines die Drainageeinrichtung enthaltenden geschlossenen Wundraums.

9. Wundbehandlungsanordnung nach einem der vorhergehenden Ansprüche, **gekennzeichnet durch** eine Saugeinrichtung zum Erzeugen eines Unterdrucks im Bereich mindestens einer äußeren Begrenzungsfläche der Drainageeinrichtung.

10. Wundbehandlungsanordnung nach Anspruch 9, **dadurch gekennzeichnet, dass** die Saugeinrichtung einen mit der Drainageeinrichtung, insbesondere der ersten Drainageschicht, fluidleitend verbundenen Drainageschlauch aufweist, der andererseits mit einer Unterdruckquelle verbunden ist.

11. Wundbehandlungsanordnung nach einem der vorhergehenden Ansprüche, **dadurch gekennzeichnet, dass** die Drainageeinrichtung bezüglich einer parallel zu einer äußeren Begrenzungsfläche verlaufenden Wickelachse aufgewickelt ist.

12. Wundbehandlungsanordnung nach einem der vorhergehenden Ansprüche, **dadurch gekennzeichnet, dass** die zweite Drainageschicht die erste Drainageschicht im Bereich ihres Rands zumindest abschnittweise in einer parallel zur zweiten äußeren Begrenzungsfläche verlaufenden Richtung überragt.

13. Verwendung einer Drainageeinrichtung mit einer mehrschichtigen Verbundstruktur zur Herstellung einer Wundbehandlungsanordnung nach einem der vorhergehenden Ansprüche.

## Claims

1. Wound treatment arrangement having a drainage device designed for draining fluids from a human or animal body, in particular from a wound, wherein the drainage device has a multilayer composite structure having two boundary surfaces which are opposite one another, are at least partially fluid-permeable, in particular run approximately parallel to one another, of which one is formed at least partially by a first outer boundary surface, facing away from a boundary layer between two adjacent drainage layers, of a first drainage layer having a first surface finish, and the other is formed at least partially by a second outer boundary surface, facing away from a boundary layer between two adjacent drainage layers, of a second drainage layer having a second surface finish which differs from the first surface finish, wherein the drainage layers are preferably connected to one another in a fluid-conducting manner, the first drainage layer has an open-pore foam, in particular polyurethane foam, and the second drainage layer has a single-layer or multilayer drainage film, wherein the second outer boundary surface is formed by a film layer which is penetrated by a plurality of perforation openings, **characterized in that** the second drainage layer is penetrated by at least one perforation hole through which the adjacent drainage layer, in particular the first drainage layer, is accessible, and at least one perforation hole has an opening area in a plane extending parallel to the second boundary surface of 10 mm² or more, in particular 25 mm² or more and/or 1000 mm² or less, in particular 400 mm² or less.

2. Wound treatment arrangement according to Claim 1, **characterized in that** the mean pore size of the open-pore foam is 200 µm or more and/or 800 µm or less.

3. Wound treatment arrangement according to one of the preceding claims, **characterized in that** the thickness of the first drainage layer in a thickness direction extending perpendicularly to the first outer boundary surface is 10 mm or more and/or 30 mm or less.

4. Wound treatment arrangement according to one of the preceding claims, **characterized in that** the second drainage layer has two film layers, between which a drainage space is formed and each of which has a plurality of perforation openings which open into the drainage space.

5. Wound treatment arrangement according to one of the preceding claims, **characterized in that** the perforation openings have an area of 0.01 mm² or more and/or 1 mm² or less in a plane extending parallel to the second outer boundary surface.

6. Wound treatment arrangement according to one of the preceding claims, **characterized in that** the second drainage layer has at least two perforation holes with different opening areas and/or opening shapes.

7. Wound treatment arrangement according to one of the preceding claims, **characterized in that** the second drainage layer has a plurality of perforation holes and the mutually facing edges of at least two perforation holes, in particular of all the perforation holes, have a spacing of 1 mm or more, preferably 5 mm or more.

8. Wound treatment arrangement according to one of the preceding claims, **characterized by** an occlusion film for producing a closed wound space containing the drainage device.

9. Wound treatment arrangement according to one of the preceding claims, **characterized by** a suction device for generating a negative pressure in the region of at least one outer boundary surface of the drainage device.

10. Wound treatment arrangement according to Claim 9, **characterized in that** the suction device has a drainage hose which is connected in a fluid-conducting manner to the drainage device, in particular to the first drainage layer, and which is connected on the other side to a negative pressure source.

11. Wound treatment arrangement according to one of the preceding claims, **characterized in that** the drainage device is wound up with respect to a winding axis running parallel to an outer boundary surface.

12. Wound treatment arrangement according to one of the preceding claims, **characterized in that** the second drainage layer projects beyond the first drainage layer in the region of its edge at least in sections in a direction running parallel to the second outer boundary surface.

13. Use of a drainage device with a multilayer composite structure for producing a wound treatment arrangement according to one of the preceding claims.

## Revendications

1. Dispositif de traitement de plaie avec un dispositif de drainage conçu pour évacuer des fluides d'un corps humain ou animal, en particulier d'une plaie, le dispositif de drainage présentant une structure composite multicouche avec deux surfaces de délimitation opposées l'une à l'autre, au moins en partie perméables aux fluides, en particulier s'étendant sensiblement parallèlement l'une à l'autre, dont l'une est formée au moins en partie par une première surface de délimitation extérieure, opposée à une couche limite entre deux couches de drainage voisines, d'une première couche de drainage présentant un premier état de surface et l'autre est formée au moins en partie par une deuxième surface de délimitation extérieure, opposée à une couche limite entre deux couches de drainage voisines, d'une deuxième couche de drainage présentant un deuxième état de surface différente de la première nature de surface, les couches de drainage étant de préférence reliées les unes aux autres de manière à conduire les fluides, la première couche de drainage présentant une mousse à pores ouverts, en particulier une mousse de polyuréthane, et la deuxième couche de drainage présentant une feuille de drainage à une ou plusieurs couches, la deuxième surface de délimitation extérieure étant formée par une couche de feuille traversée par une pluralité d'ouvertures de perforation, **caractérisé en ce que** la deuxième couche de drainage est traversée par au moins un trou de perforation à travers lequel la couche de drainage voisine, en particulier la première couche de drainage, est accessible et au moins un trou de perforation présentant une surface d'ouverture dans un plan s'étendant parallèlement à la deuxième surface de délimitation de 10 mm2 ou plus, en particulier de 25 mm² ou plus et/ou de 1 000 mm² ou moins, en particulier de 400 mm² ou moins.

2. Dispositif de traitement de plaie selon la revendication 1, **caractérisé en ce que** la taille de pore moyenne de la mousse à pores ouverts est de 200 µm ou plus et/ou de 800 µm ou moins.

3. Dispositif de traitement de plaie selon l'une quelconque des revendications précédentes, **caractérisé en ce que** l'épaisseur de la première couche de drainage dans une direction d'épaisseur s'étendant perpendiculairement à la première surface de délimitation extérieure est de 10 mm ou plus et/ou de 30 mm ou moins.

4. Dispositif de traitement de plaie selon l'une quelconque des revendications précédentes, **caractérisé en ce que** la deuxième couche de drainage présente deux couches de film, entre lesquelles est formé un espace de drainage et dont chacune présente une pluralité d'ouvertures de perforation qui débouchent dans l'espace de drainage.

5. Dispositif de traitement de plaie selon l'une quelconque des revendications précédentes, **caractérisé en ce que** les ouvertures de perforation présentent une surface de 0,01 mm² ou plus et/ou de 1 mm² ou moins dans un plan s'étendant parallèlement à la deuxième surface de délimitation extérieure.

6. Dispositif de traitement de plaie selon l'une quelconque des revendications précédentes, **caractérisé en ce que** la deuxième couche de drainage présente au moins deux trous de perforation avec des surfaces et/ou des formes d'ouverture différentes.

7. Dispositif de traitement de plaie selon l'une quelconque des revendications précédentes, **caractérisé en ce que** la deuxième couche de drainage présente une pluralité de trous de perforation et les bords tournés l'un vers l'autre d'au moins deux trous de perforation, en particulier de tous les trous de perforation, présentent une distance de 1 mm ou plus, de préférence de 5 mm ou plus.

8. Dispositif de traitement de plaie selon l'une quelconque des revendications précédentes, **caractérisé par** un film d'occlusion pour la fabrication d'un espace de plaie fermé contenant le dispositif de drainage.

9. Dispositif de traitement de plaie selon l'une quelconque des revendications précédentes, **caractérisé par** un dispositif d'aspiration pour la production d'une dépression dans la région d'au moins une surface de délimitation extérieure du dispositif de drainage.

10. Dispositif de traitement de plaie selon la revendication 9, **caractérisé en ce que** le dispositif d'aspiration présente un tuyau de drainage relié de manière fluidique au dispositif de drainage, en particulier à la première couche de drainage, qui est relié d'autre part à une source de sous-pression.

11. Dispositif de traitement de plaie selon l'une quelconque des revendications précédentes, **caractérisé en ce que** le dispositif de drainage est enroulé par rapport à un axe d'enroulement s'étendant parallèlement à une surface de délimitation extérieure.

12. Dispositif de traitement de plaie selon l'une quelconque des revendications précédentes, **caractérisé en ce que** la deuxième couche de drainage dépasse de la première couche de drainage dans la région de son bord au moins par sections dans une direction s'étendant parallèlement à la deuxième surface de délimitation extérieure.

13. Utilisation d'un dispositif de drainage avec une structure composite multicouche pour la fabrication d'un dispositif de traitement de plaie selon l'une quelconque des revendications précédentes.
